# FASCICULE DE BREVET EUROPEEN

(11) **EP 1 463 468 B1**
(45) Date de publication et mention de la délivrance du brevet: **14.09.2016**
(21) Numéro de dépôt: 03715007.5
(22) Date de dépôt: 08.01.2003
(51) Int. Cl.: A61F 5/00

(54) **ANNEAU GASTRIQUE DE TRAITEMENT DE L OBESITE**
RING ZUR VERENGUNG DES MAGENS ZUM BEHANDELN DER FETTLEIBIGKEIT
GASTRIC RING FOR THE TREATMENT OF OBESITY

(30) Priorité: 09.01.2002 FR 0200261
(43) Date de publication de la demande: 06.10.2004
(73) Titulaire: SOFRADIM PRODUCTION, 01600 Trévoux (FR)
(72) Inventeur: BAILLY, Pierre, F-69300 Caluire (FR); THERIN, Michel, F-69004 Lyon (FR)
(74) Mandataire: Brédeville, Odile Marie
(86) Numéro de dépôt international: PCT/FR2003/000040
(87) Numéro de publication internationale: WO 2003/057092

(56) Documents cités:
- EP-A- 0 263 360
- EP-A- 0 531 742
- EP-A- 0 611 561
- WO-A-86/04498

## Description

La présente invention concerne un anneau gastrique de traitement de l'obésité. Un tel anneau est également couramment dénommé "anneau de gastroplastie".

Il est connu de traiter l'obésité pathologique d'un patient en plaçant un anneau autour de l'estomac de ce patient, de manière à créer, sur la partie supérieure de l'estomac, une poche de dimensions restreintes et une ouverture d'écoulement des aliments ayant également des dimensions restreintes.

De tels anneaux sont bien connus dans leur principe, et les documents WO-A-86/04498 ou EP-A-0 611 561 peuvent être cités en tant que documents illustrant des anneaux gastriques existants.

Certains des anneaux existants ont pour inconvénient d'être relativement agressifs pour la paroi de l'estomac, au point de provoquer des inflammations de cette paroi, voire, dans des cas extrêmes, des perforations de celle-ci. Cette agressivité résulte de l'aspect massif et rigide de cet anneau et de la présence d'une poche gonflable située sur la face interne de l'anneau, cette poche gonflable permettant d'ajuster la surface de l'ouverture délimitée par l'anneau en exerçant sur la paroi gastrique une pression dirigée radialement vers l'intérieur.

Les chambres implantables permettant, par voie percutanée, de gonfler ou de dégonfler de telles poches gonflables, ainsi que les tubulures reliant ces chambres et ces poches, ont pour inconvénient de présenter des risques de fuite, de migration et d'infection.

Les anneaux existants ont également pour inconvénient de présenter des risques de bascule ou de glissement appelé "slippage" et de nécessiter des ré-opérations lorsqu'il est nécessaire de les replacer ou de les retirer après une certaine durée de traitement.

Le document EP 0 628 292 A1 décrit un implant de forme cylindrique apte à former un anneau que l'on peut fermer par engagement d'une extrémité de l'implant dans un oeillet situé à l'autre extrémité de l'implant.

La présente invention vise à remédier à l'ensemble de ces inconvénients des dispositifs existants, par des dispositions ou moyens pouvant se compléter les uns les autres.

L'anneau qu'elle concerne comprend, de manière connue en soi,
- une bande propre à former un anneau autour de la paroi de l'estomac, et
- des moyens d'assemblage permettant de maintenir cette bande sous forme dudit anneau.

La bande est constituée en au moins un matériau biorésorbable ou biodégradable, et comporte une face destinée à venir au contact de la paroi de l'estomac.

Par " biodégradable" ou "biorésorbable", on entend la propriété selon laquelle un matériau se dégrade in vivo par un mécanisme cellulaire, enzymatique ou microbien (cf. par exemple dégradation du collagène par la collagènase), ou par un mécanisme physico-chimique (cf. par exemple hydrolyse d'un polymère d'acide lactique).

Un tel matériau biorésorbable est de préférence choisi dans le groupe consistant en les polymères de p-dioxanone, les polyglycolides, les polyorthoesters, les polymères de triméthylène carbonate, les stéréocopolymères de l'acide L et D lactique, les homopolymères de l'acide L lactique, les copolymères de l'acide lactique et d'un comonomère compatible, tels que les dérivés d'alpha-hydroxy acides. De manière encore plus préférée, le matériau biorésorbable présente une polydispersité inférieure à 2.

A titre d'exemple préféré, le matériau biodégradable ou biorésorbable est un polymère d'acide lactique (PLA) ou polyglycolique (PGA), ou un copolymère d'acide lactique ou polyglycolique (PLA-PGA).

Préférentiellement, la bande comprend ou est constituée en au moins un matériau biorésorbable.

La biorésorbabilité de la bande élimine la nécessité d'une ablation de l'anneau et donc d'avoir à réopérer le patient à cette fin. De plus et surtout, la fibrose qui se constitue naturellement autour de cette bande est conservée et subsiste après la complète résorption de la bande, cette fibrose engendrant une contraction cicatricielle de la paroi gastrique, qui rend inutile d'utiliser une poche gonflable pour ajuster la section de l'ouverture de l'anneau.

L'intégration tissulaire progressive de la bande solidarise l'anneau à la paroi de l'estomac et permet une parfaite prévention de la migration ("slippage") de l'anneau et l'obtention d'une large surface de contact des tissus avec la bande, favorisant la résorption de cette dernière.

La bande peut être constituée par un polymère d'acide lactique ou polyglycolique, ou par un copolymère d'acide lactique ou polyglycolique.

Ces matériaux présentent une cinétique de résorption sur plusieurs mois voire plusieurs années, apte à maintenir l'effet de la bande sur une période suffisante pour réduire de façon significative l'index de masse corporelle.

La bande selon l'invention peut être obtenue selon toute technique appropriée, notamment par moulage ou par extrusion de son matériau constitutif, par exemple thermo-plastique.

De préférence, au moins la face de la bande destinée à venir au contact de la paroi de l'estomac comporte un revêtement lisse propre à séparer cette face et cette paroi au moins temporairement.

Ce revêtement permet d'empêcher ou de différer le contact direct entre la bande et la paroi de l'estomac le temps que les phases les plus inflammatoires de la cicatrisation de cette paroi soient passées. L'intégration tissulaire précitée est donc dissociée dans le temps par rapport à la cicatrisation des lésions initiales générées par la mise en place de l'anneau, ce qui limite ainsi le risque d'érosion de la paroi gastrique.

Ce revêtement peut être en un matériau biorésorbable, notamment un matériau collagénique réticulé.

La bande a une épaisseur telle qu'elle présente une souplesse lui permettant d'être courbée transversalement à sa direction longitudinale. Lesdits moyens d'assemblage que comprend l'anneau selon l'invention incluent deux portions latérales de la bande, qui prolongent la portion médiane de la bande destinée à entourer l'estomac du patient, et sont conformés pour placer lesdites portions latérales de la bande selon une direction sensiblement radiale par rapport au cercle que forme la portion médiane de la bande lorsque l'anneau est mis en place sur l'estomac d'un patient, ces portions latérales faisant saillie vers l'extérieur de ce cercle.

L'anneau gastrique n'implique alors pas de chevauchement de l'une de ces portions latérales l'une sur l'autre, un tel chevauchement conduisant, sur certains dispositifs de l'art antérieur, à créer une surépaisseur au niveau de la face radialement interne dudit cercle, qui est agressive pour la paroi de l'estomac.

Selon une forme de réalisation possible de l'invention, lesdits moyens d'assemblage incluent au moins une attache comprenant des parties mobiles propres à venir en prise avec lesdites portions latérales de la bande.

Avantageusement, dans ce cas, lesdites parties mobiles sont déplaçables entre deux positions, à savoir une position de coulissement, dans laquelle l'attache peut coulisser le long desdites portions latérales, et une position de verrouillage desdites portions latérales, dans laquelle toute possibilité de coulissement de l'attache par rapport à ces portions latérales est empêchée.

Le coulissement de l'attache le long desdites portions latérales permet d'ajuster le serrage de la bande autour de l'estomac.

Lesdites portions latérales de la bande peuvent alors présenter une longueur supérieure à ce qui est nécessaire pour la prise d'appui de l'attache sur elles, cette longueur étant telle que l'attache peut être coulissée le long de ces portions latérales jusqu'à obtention de la section désirée pour l'ouverture délimitée par l'anneau, et être ensuite amenée en position de verrouillage de ces portions latérales.

L'attache peut comprendre des moyens d'immobilisation desdites parties mobiles dans les positions respectives précitées de coulissement et de verrouillage, de préférence de type à encliquetage irréversible.

L'attache peut comprendre ou être constituée en un matériau biorésorbable ou biodégradable. Elle peut inclure un produit radio-opaque de type sulfate de barium lorsqu'elle n'est pas en un matériau radio-opaque par nature.

Selon une possibilité, l'attache comprend deux parties mobiles dimensionnées pour entourer lesdites portions latérales de la bande et maintenir ces portions serrées l'une contre l'autre, ces parties mobiles étant reliées l'une à l'autre par l'une de leurs extrémités longitudinales, au moyen d'une charnière-film.

Cette charnière-film peut alors être conformée de manière à maintenir normalement lesdites parties mobiles dans une position d'éloignement d'une partie mobile par rapport à l'autre, afin de faciliter l'engagement de l'attache sur lesdites portions latérales de la bande.

L'une desdites parties mobiles peut comprendre au moins une nervure faisant saillie de sa face tournée vers l'autre partie mobile, cette autre partie mobile présentant alors une lumière dans laquelle la ou les nervures sont destinées à être engagées.

Selon une autre possibilité, l'attache est en un matériau non élastiquement déformable, tel qu'un matériau métallique, et comprend des pattes déformables susceptibles, dans une position de déformation, de pénétrer dans le matériau qui forme lesdites portions latérales de la bande et d'assurer ainsi la fixation de ces portions latérales l'une par rapport à l'autre.

Une telle attache en un matériau métallique a pour avantage d'être peu encombrante.

Selon une disposition non conforme à l'invention, la bande de l'anneau peut également avoir une épaisseur telle qu'elle présente une rigidité empêchant qu'elle soit courbée transversalement à sa direction longitudinale.

Dans ce cas, la bande peut être en au moins deux parties reliées de manière pivotante l'une à l'autre, permettant de placer cette bande selon une forme allongée telle que cette bande peut être introduite dans le corps du patient par des techniques de chirurgie peu invasives comme la ccelioscopie ou la laparoscopie.

La bande peut notamment comprendre une pluralité de "maillons" reliés de manière pivotante les uns aux autres.

Lesdits moyens d'assemblage que comprend l'anneau peuvent alors comprendre une patte présentant au moins un cran, pouvant être engagée dans une cavité correspondante dans laquelle fait saillie au moins un cran complémentaire de celui de la patte, le cran de la patte pouvant dépasser le cran de la cavité lors de l'engagement de la patte dans la cavité et être verrouillé derrière celui-ci pour assurer l'assemblage des deux extrémités de la bande l'une à l'autre.

Pour sa bonne compréhension, l'invention est à nouveau décrite ci-dessous en référence au dessin schématique annexé représentant, à titre d'exemples non limitatifs, trois formes de réalisation possibles de l'anneau gastrique qu'elle concerne.

Par simplification, les éléments ou partie d'éléments qui se retrouvent d'une forme de réalisation à l'autre sont désignés par les mêmes références numériques.
La figure 1 en est une vue en perspective d'un anneau gastrique selon une première forme de réalisation, alors qu'il est mis en place sur l'estomac d'un patient ;
la figure 2 en est une vue en coupe selon la ligne II-II de la figure 1;
la figure 3 est une vue à échelle agrandie d'une attache qu'il comprend, en coupe selon la ligne III-III de la figure 4, et en position d'ouverture de cette attache ;
la figure 4 est une vue de l'attache en coupe selon la ligne IV-IV de la figure 3 ;
la figure 5 est une vue de l'attache similaire à la figure 3, dans une position de fermeture partielle de cette attache, permettant un coulissement de cette attache par rapport à des portions latérales d'une bande que comprend l'anneau ;
la figure 6 est une vue de l'attache similaire à la figure 4, dans cette même position de fermeture partielle ;
la figure 7 est une vue de l'attache similaire à la figure 3, dans une position de fermeture totale de cette attache, interdisant toute possibilité de coulissement de l'attache par rapport aux portions latérales de ladite bande ;
la figure 8 est une vue de l'attache similaire à la figure 4, dans cette même position de fermeture totale ;
la figure 9 est une vue de côté de l'attache précitée, selon une deuxième forme de réalisation ;
la figure 10 est une vue de cette attache selon une direction opposée à celle selon la figure 9 ;
la figure 11 est une vue de cette attache en coupe selon la ligne XI-XI de la figure 9, l'attache étant en position d'ouverture ;
la figure 12 est une vue de l'attache similaire à la figure 11, l'attache étant en position de fermeture partielle ;
la figure 13 est une vue de l'attache similaire à la figure 11, l'attache étant en position de fermeture totale ;
la figure 14 est une vue de côté de l'anneau selon une forme de réalisation non conforme à l'invention, avant fermeture ;
la figure 15 en est une vue de dessus ;
la figure 16 en est une vue de côté, après fermeture, et
la figure 17 est une vue de détail de la figure 16.

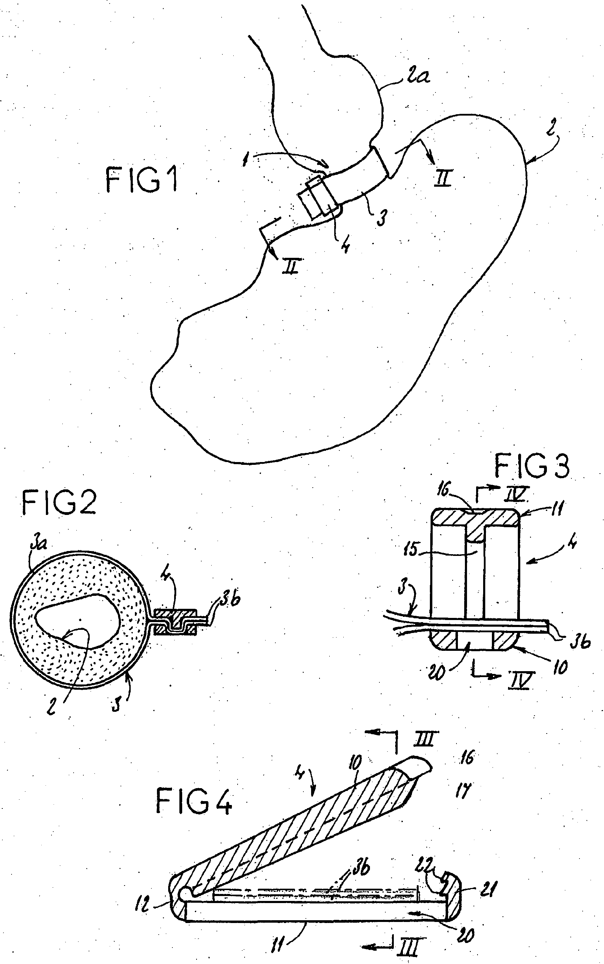

La figure 1 représente un anneau gastrique 1 de traitement de l'obésité pathologique d'un patient, mis en place sur l'estomac 2 de ce patient.

Cet anneau 1 comprend une bande 3 et une attache 4 propre à maintenir la bande 3 autour de l'estomac 2, afin de créer, sur la partie supérieure de l'estomac, une poche 2a de dimensions restreintes et une ouverture distale d'écoulement des aliments ayant également des dimensions restreintes.

Dans cette forme de réalisation de l'anneau, la bande 3 a une épaisseur telle qu'elle présente une souplesse lui permettant d'être courbée transversalement à sa direction longitudinale ; comme le montre la figure 2, elle définit une portion médiane 3a propre à entourer la paroi de l'estomac 2 selon un parcours circulaire et deux portions latérales 3b propres à être assemblées l'une à l'autre au moyen de l'attache 4, pour maintenir la bande 3 autour de l'estomac 2.

Les portions 3b présentent une longueur bien supérieure à ce qui est nécessaire pour la prise d'appui de l'attache 4 sur elles. Elles permettent ainsi une manipulation aisée de la bande 3, et notamment son passage rétrogastrique.

La bande 3 est en un matériau biorésorbable ou biodégradable, tel qu'un polymère d'acide lactique ou polyglycolique, ou un copolymère d'acide lactique ou polyglycolique, et a été obtenue par moulage ou par extrusion.

En référence aux figures 3 à 8, il apparaît que l'attache 4 comprend deux parties mobiles 10, 11 dimensionnées pour entourer les portions latérales 3b de la bande 3 et maintenir ces portions 3b serrées l'une contre l'autre selon une direction sensiblement radiale par rapport au cercle que forme la portion médiane 3a, comme le montre la figure 2, ces portions latérales 3b faisant saillie vers l'extérieur de ce cercle.

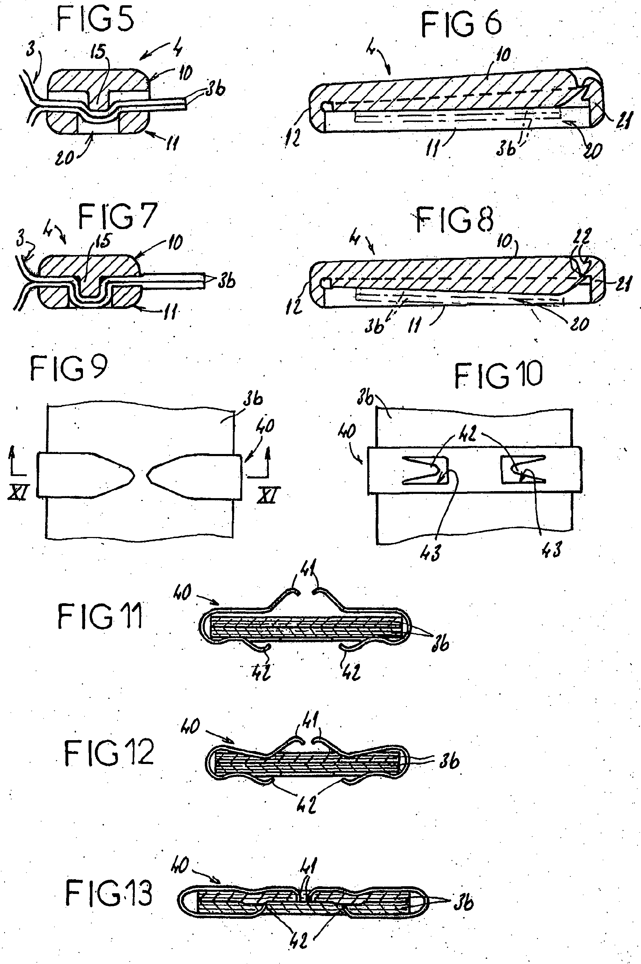

Les parties mobiles 10, 11 sont reliées l'une à l'autre par l'une de leurs extrémités longitudinales, au moyen d'une charnière-film 12. Cette charnière-film 12, à l'état non déformée, maintient les parties 10, 11 dans la position montrée sur les figures 3 et 4, dans laquelle la partie 10 est éloignée de la partie 11. Cet éloignement facilite l'engagement de l'attache 4 sur les portions 3b, particulièrement lorsque cette attache 4 est mise en place par des techniques peu invasives tel que coelioscopie ou laparoscopie. La charnière-film 12 peut être déformée pour permettre à la partie 10 de venir dans les positions de fermeture partielle et de fermeture totale de l'attache 4, représentées sur les figures 5 et 6, et 7 et 8, respectivement.

La partie 10 comprend une nervure 15 faisant saillie de sa face tournée vers la partie 11 et une encoche médiane 16 aménagée dans son bord latéral d'extrémité, du côté opposé à la charnière 12. La nervure 15 forme, au niveau du fond de l'encoche 16, un cran d'encliquetage 17, c'est-à-dire une saillie présentant une paroi inclinée du côté de la partie 11 et une paroi plane de verrouillage du côté opposé à cette partie 11.

La partie 11 présente une lumière centrale 20 dans laquelle la nervure 15 est destinée à être engagée, comme le montrent les figures 7 et 8. Du côté opposé à la charnière 12, cette partie 11 comprend une dent médiane 21 propre à être engagée dans l'encoche 16, qui est équipée de deux crans d'encliquetage étagés 22. Ces crans 22 sont propres à coopérer avec le cran 17 comme le montrent respectivement les figures 6 et 8.

Ainsi que cela se comprend en référence aux figures 3 à 8, ces crans 17 et 22 forment des moyens à encliquetage irréversible permettant de verrouiller lesdites parties mobiles 10, 11 l'une par rapport à l'autre selon deux positions, à savoir :
- une position de non-serrage des portions latérales 3b, montrée sur les figures 5 et 6, dans laquelle ces deux portions 3b peuvent coulisser avec frottements entre la nervure 15 et les portions de la partie 11 qui délimitent longitudinalement la lumière 20 ; et
- une position de verrouillage de ces portions latérales 3b, montrée sur les figures 7 et 8 dans laquelle toute possibilité de coulissement de ces portions 3b est empêchée, du fait du serrage de ces portions 3b entre la nervure 15 et lesdites portions de la partie 11 qui délimitent longitudinalement la lumière 20.

L'attache 4 peut ainsi être mise en place sur les portions 3b alors qu'elle est en position d'ouverture, être amenée en position de non-serrage par simple pression de la partie 10 en direction de la partie 11 de manière à amener le cran 17 en prise avec le cran 22 supérieur, être coulissée le long de ces portions 3b jusqu'à obtention de la section désirée pour l'ouverture d'admission de nourriture dans l'estomac 2, et être amenée en position de verrouillage par simple pression de la partie 10 en direction de la partie 11 de manière à amener le cran 17 en prise avec le cran 22 inférieur. Les parties en excès des portions 3b peuvent être ensuite sectionnées.

Les figures 9 à 13 montrent une attache 40 selon une deuxième forme de réalisation, formée par pliage d'une bande métallique selon une forme de "C", de manière à délimiter un conduit dans lequel les portions 3b peuvent être engagées.

Cette attache 40 comprend, sur un côté, deux pattes recourbées 41 formées dans les extrémités de ladite bande métallique et, sur le côté opposé, deux pattes recourbées 42 individualisées par des découpes appropriées 43 de cette même bande métallique. Les pattes 41 sont susceptibles de pénétrer dans l'une des portions 3b tandis que les pattes 42 sont susceptibles de pénétrer dans l'autre portion 3b.

Comme le montre la figure 12, l'attache 40 peut être déformée en dehors des pattes 41, 42 pour l'obtention d'une possibilité de coulissement des portions 3b avec frottements dans l'attache 40.

La figure 13 montre que cette même attache 40 peut être déformée au niveau des pattes 41, 42 pour réaliser une pénétration de ces pattes 41, 42 dans les portions 3b afin de bloquer toute possibilité de coulissement de ces portions 3b dans l'attache 40.

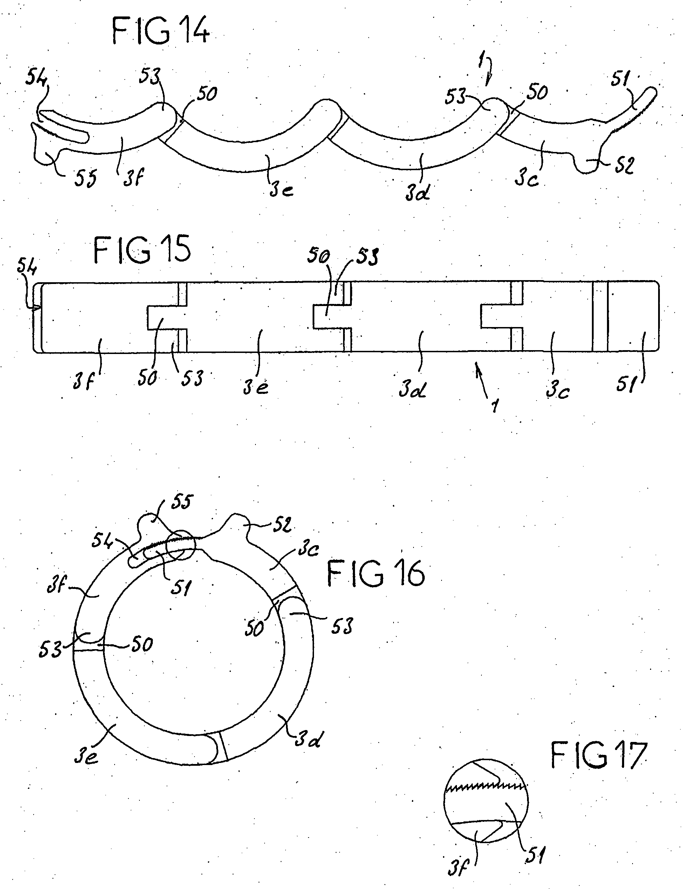

Les figures 14 à 16 montrent un anneau 1 selon une forme de réalisation non conforme à l'invention, dans laquelle la bande 3 a une épaisseur telle qu'elle présente une rigidité empêchant qu'elle soit courbée transversalement à sa direction longitudinale.

La bande 3 comprend alors quatre "maillons" 3c, 3d, 3e, 3f reliés de manière pivotante les uns aux autres, qui permettent de placer cette bande 3 selon la forme allongée montrée sur les figures 14 et 15. Cette forme allongée est telle que la bande 3 peut être introduite dans le corps du patient par des techniques de chirurgie peu invasives comme la coelioscopie ou la laparoscopie.

Le maillon 3c situé à une extrémité de la bande 3 comprend, à une extrémité, un tenon 50 permettant son assemblage, avec possibilité de pivotement, au maillon adjacent 3d, et, à son autre extrémité, une patte courbe 51 comprenant une pluralité de crans en dents de loup. Sur sa face convexe et près de la patte 51, le maillon 3c présente un bossage 52.

Le maillon 3d comprend, à son extrémité tournée vers le maillon 3c, deux pattes 53 formant une chape de réception du tenon 50 et, à son extrémité, un tenon 50 identique à celui du maillon 3c. L'assemblage des pattes 53 et du tenon 50 correspondant est réalisé au moyen d'un axe, non représenté.

Le maillon 3e comprend des pattes 53 et un tenon 50 identiques à ceux du maillon 3d.

Le maillon 3f situé à l'autre extrémité de la bande 3 comprend, à une extrémité, des pattes 53 identiques à celles du maillon 3d, et, à son autre extrémité, une cavité 54 de forme correspondant à celle de la patte 51, dans laquelle font saillie une pluralité de crans en dents de loup complémentaires de ceux de la patte 51. Sur sa face convexe et sur un côté de la zone qui délimite la cavité 54, le maillon 3f présente un bossage 55 identique au bossage 52.

Comme le montrent les figures 16 et 17, les crans de la patte 51 viennent en prise avec les crans de la cavité 54 et se verrouillent avec ceux-ci pour assurer l'assemblage des deux extrémités de la bande 3 l'une à l'autre. Les bossages 52 et 55 permettent l'appui d'un outil permettant le coulissement de la patte 51 dans la cavité 54.

Ainsi qu'il apparaît de ce qui précède, l'invention apporte une amélioration déterminante à la technique antérieure, en fournissant un anneau gastrique peu agressif pour la paroi de l'estomac, facile à mettre en place sur l'estomac du patient, et ne nécessitant pas une ablation en fin de traitement.

Cet anneau a particulièrement pour avantage de permettre de conserver la fibrose qui se constitue naturellement autour de la bande 3, cette fibrose engendrant une contraction cicatricielle de la paroi gastrique, qui rend inutile d'utiliser une poche gonflable pour ajuster la section de l'ouverture de l'anneau.

L'intégration tissulaire progressive de la bande solidarise l'anneau à la paroi de l'estomac et permet une parfaite prévention de la migration ("slippage") de l'anneau et l'obtention d'une large surface de contact des tissus avec la bande, favorisant la résorption de cette dernière.

## Revendications

1. - Anneau gastrique (1) de traitement de l'obésité, comprenant:
- une bande (3) propre à former un anneau autour de la paroi de l'estomac, et
- des moyens d'assemblage (4, 40, 51, 54) permettant de maintenir cette bande sous forme dudit anneau ;
la bande étant constituée en au moins un matériau biorésorbable ou biodégradable, et comportant une face destinée à venir au contact de la paroi de l'estomac (2),
**caractérisé en ce que** la bande (3) a une épaisseur telle qu'elle présente une souplesse lui permettant d'être courbée transversalement à sa direction longitudinale, et **en ce que** lesdits moyens d'assemblage (4, 40) incluent deux portions latérales (3b) de la bande (3), qui prolongent la portion médiane (3a) de la bande (3) destinée à entourer l'estomac (2) du patient, et sont conformés pour placer lesdites portions latérales (3b) de la bande (3) selon une direction sensiblement radiale par rapport au cercle que forme la portion médiane (3a) de la bande (3) lorsque l'anneau (1) est mis en place sur l'estomac (2) d'un patient, ces portions latérales (3b) faisant saillie vers l'extérieur de ce cercle.

2. - Anneau gastrique selon la revendication 1, **caractérisé en ce qu'**au moins la face dé la bande (3) destinée à venir au contact de la paroi de l'estomac (2) comporte un revêtement lisse propre à séparer cette face et cette paroi au moins temporairement.

3. - Anneau gastrique selon la revendication 2, **caractérisé en ce que** ledit revêtement est en un matériau biorésorbable ou biodégradable, notamment un matériau collagénique réticulé.

4. - Anneau gastrique selon la revendication 1, **caractérisé en ce que** les moyens d'assemblage comprennent ou sont constitués en au moins un matériau biorésorbable ou biodégradable.

5. - Anneau gastrique selon l'une des revendications 1-4, **caractérisé en ce que** lesdits moyens d'assemblage incluent au moins une attache (4, 40) comprenant des parties mobiles (10, 11 ; 41, 42) propres à venir en prise avec lesdites portions latérales (3b) de la bande (3).

## Patentansprüche

1. Magenring (1) zur Behandlung von Adipositas, der Folgendes umfasst:
- ein Band (3), das dazu geeignet ist, einen Ring um die Wand des Magens herum zu bilden, und
- Zusammenfügungsmittel (4, 40, 51, 54), die das Halten dieses Bands in der Form des besagten Rings ermöglichen;
wobei das Band aus mindestens einem bioresorbierbaren oder biologisch abbaubaren Material besteht und eine Seite umfasst, die dazu vorgesehen ist, mit der Wand des Magens (2) in Kontakt zu kommen,
**dadurch gekennzeichnet, dass** das Band (3) eine derartige Dicke hat, dass es eine Flexibilität aufweist, die ermöglicht, es in seiner Längsrichtung quer zu biegen, und dass die besagten Zusammenfügungsmittel (4, 40) zwei seitliche Abschnitte (3b) des Bands (3) beinhalten, die den mittleren Abschnitt (3a) des Bands (3) verlängern, der dazu vorgesehen ist, den Magen (2) des Patienten zu umschließen, und zum Anordnen der besagten seitlichen Abschnitte (3b) des Bands (3) gemäß einer im Wesentlichen radialen Richtung in Bezug auf den Kreis angepasst sind, der von dem mittleren Abschnitt (3a) des Bands (3) gebildet wird, wenn der Ring (1) auf dem Magen (2) eines Patienten platziert wird, wobei diese seitlichen Abschnitte (3b) zur Außenseite dieses Kreises hervorstehen.

2. Magenring nach Anspruch 1, **dadurch gekennzeichnet, dass** mindestens die Seite des Bands (3), die dazu vorgesehen ist, mit der Wand des Magens (2) in Kontakt zu kommen, eine glatte Beschichtung umfasst, die dazu geeignet ist, diese Seite und diese Wand zumindest vorübergehend zu trennen.

3. Magenring nach Anspruch 2, **dadurch gekennzeichnet, dass** die besagte Beschichtung aus einem bioresorbierbaren oder biologisch abbaubaren Material, insbesondere einem vernetzten kollagenhaltigen Material ist.

4. Magenring nach Anspruch 1, **dadurch gekennzeichnet, dass** die Zusammenfügungsmittel mindestens ein bioresorbierbares oder biologisch abbaubares Material umfassen oder aus diesem bestehen.

5. Magenring nach einem der Ansprüche 1 bis 4, **dadurch gekennzeichnet, dass** die besagten Zusammenfügungsmittel mindestens eine Klammer (4, 40) beinhalten, die bewegliche Teile (10, 11; 41, 42) umfasst, die dazu geeignet sind, in Eingriff mit den besagten seitlichen Abschnitten (3b) des Bands (3) zu kommen.

## Claims

1. A gastric ring (1) for treatment of obesity, comprising:
- a band (3) which is able to form a ring around the wall of the stomach, and
- connection means (4, 40, 51, 54) with which this band can be maintained in the form of said ring;
the band being made of at least one bioabsorbable or biodegradable material and having a face intended to come into contact with the wall of the stomach (2),
**characterized in that** the band (3) is of such a thickness that it has a flexibility allowing it to be curved transversely with respect to its longitudinal direction, and **in that** said connection means (4, 40) include two lateral portions (3b) of the band (3) which continue the central portion (3a) of the band (3) intended to surround the patient's stomach (2) and are designed for placing said lateral portions (3b) of the band (3) in a substantially radial direction with respect to the circle formed by the central portion (3a) of the band (3) when the ring (1) is placed on the stomach (2) of a patient, these lateral portions (3b) protruding outward from this circle.

2. The gastric ring as claimed in claim 1, **characterized in that** at least the face of the band (3) intended to come into contact with the wall of the stomach (2) has a smooth coating for separating this face and this wall at least temporarily.

3. The gastric ring as claimed in claim 2, **characterized in that** said coating is made of a bioabsorbable or biodegradable material, in particular a crosslinked collagen material.

4. The gastric ring as claimed in claim 1, **characterized in that** the connection means comprise or are made of at least one bioabsorbable or biodegradable material.

5. The gastric ring as claimed in any one of claims 1-4, **characterized in that** said connection means include at least one fastener (4, 40) comprising movable parts (10, 11 ; 41, 42) able to engage with said lateral portions (3b) of the band (3).
